**Europäisches Patentamt**

**European Patent Office**

**Office européen des brevets**

(19)

(11) Veröffentlichungsnummer: **0 051 204**
A1

(12) **EUROPÄISCHE PATENTANMELDUNG**

(21) Anmeldenummer: 81108535.6

(22) Anmeldetag: 20.10.81

(51) Int. Cl.³: **C 07 C 103/52**
**A 61 K 37/02, A 61 K 49/02**
**G 01 N 33/54**

(30) Priorität: 30.10.80 DE 3040824

(43) Veröffentlichungstag der Anmeldung:
12.05.82 Patentblatt 82/19

(84) Benannte Vertragsstaaten:
AT BE CH DE FR GB IT LI LU NL SE

(71) Anmelder: Dr. Karl Thomae GmbH
Postfach 1755
D-7950 Biberach (Riss)(DE)

(72) Erfinder: Jung, Günther, Dipl.-Chem. Prof. Dr.
Ob der Grafenhalde 5
D-7400 Tübingen 1(DE)

(72) Erfinder: Brückner, Hans, Dipl.-Chem. Dr.
Lindenstrasse 55
D-7302 Ostfieldern/Nellingen(DE)

(72) Erfinder: Swetly, Peter, Dr.
Hietzinger Hauptstrasse 40 B
A-1130 Wien(AT)

(72) Erfinder: Bozler, Gerhard, Dipl.-Chem. Dr.
Alpenstrasse 38
D-7950 Biberach 1(DE)

(54) Neues Dekapeptid, Verfahren zu seiner Herstellung und Verwendung.

(57) Neues Dekapeptid der Formel H-Ser-Asp-Leu-Pro-Gln-Thr-His-Ser-Leu-Gly-OH

und dessen Verwendung als Hapten, Tracer oder Antikörper.
Das neue Peptid kann nach für analoge Verbindungen
bekannten Verfahren hergestellt werden.

EP 0 051 204 A1

COMPLETE DOCUMENT

Dr. Karl Thomae GmbH
Case 5/799
Dr. So./Kp.

Neues Dekapeptid, Verfahren zu seiner Herstellung
und Verwendung

Die vorliegende Erfindung betrifft ein neues Dekapeptid, nämlich H-Ser-Asp-Leu-Pro-Gln-Thr-His-Ser-Leu-Gly-OH, ein Verfahren zu seiner Herstellung nach der Festphasen-Synthese und
seine Verwendung, beispielsweise als Hapten zur Kupplung mit
einem Immunogen.

Hochgereinigtes Interferon, das weltweit als hochinteressante
Substanz bearbeitet wird, steht bis heute nur in äußerst geringen Mengen für medizinische Untersuchungen zur Verfügung. Es
war daher bisher nicht möglich, an Interferonproteinen strukturelle oder biochemische Untersuchungen durchzuführen. Nachdem
nunmehr die N-terminale Sequenz des menschlichen Lymphoblasteninterferons aufgeklärt werden konnte (Science 207, 527 (1980)),
wurde das obige Dekapeptid erstmalig synthetisiert und seine
Eigenschaften untersucht. Diese Aminosäuresequenz läßt sich
ebenfalls aus der Deoxyribonukleinsäuresequenz des 5'-terminalen Endes des Interferon-Strukturgens oder der Ribonukleotidsequenz des 5'-Endes der Translationseinheit der Interferonmessenger Ribonukleinsäure, beginnend mit dem Initiationstriplet A.U.G. (A = Adenosin, U - Uridin, G = Guanosin) nach der
Regel des genetischen Codes ableiten.

Im Nachfolgenden werden folgende in der Peptidchemie übliche Abkürzungen benützt:

| H-Ser-OH | = | L-Serin |
| H-Asp-OH | = | L-Asparaginsäure |
| H-Leu-OH | = | L-Leucin |
| H-Pro-OH | = | L-Prolin |
| H-Glu-OH | = | L-Glutaminsäure |
| H-Thr-OH | = | L-Threonin |
| H-His-OH | = | L-Histidin |
| H-Gly-OH | = | Glycin |

| Bzl | = | Benzylrest, |
| Dnp | = | 2,4-Dinitrophenylrest, |
| Boc | = | tert.Butyloxycarbonylrest, |
| Fmoc | = | 9-Fluorenylmethyloxycarbonylrest |
| $Bu^t$ | = | tert.Butylrest |
| DMF | = | Dimethylformamid |
| DCC | = | N,N'-Dicyclohexylcarbodiimid |
| HOBt | = | 1-Hydroxybenzotriazol |

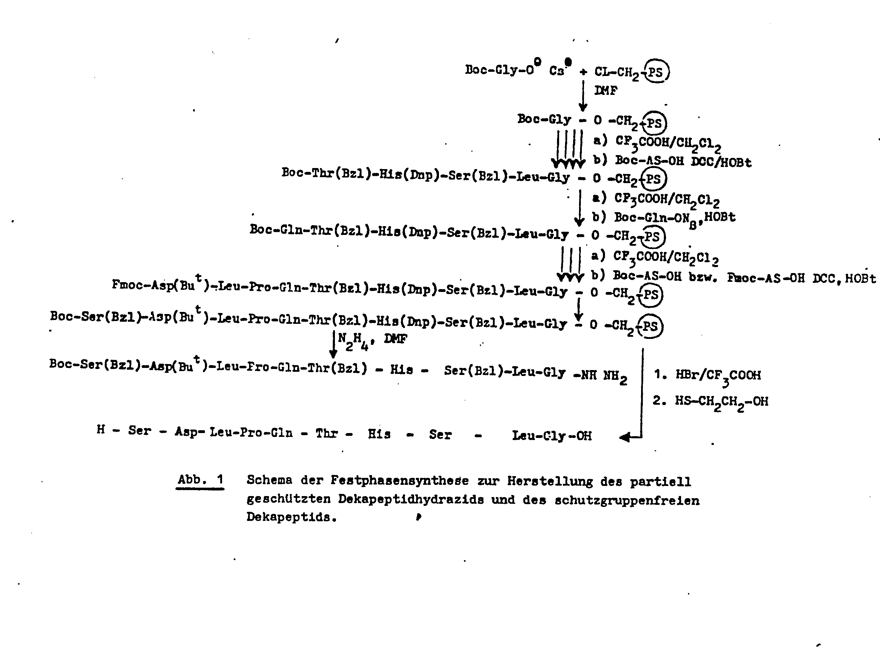

Die Herstellung des neuen Dekapeptids erfolgt nach der Festphasen-Synthese (siehe R. B. Merrifield in J. Amer. chem. Soc.
85, 2149-2154 (1963)). Bei dieser wird die C-terminale Aminosäure eines Peptids über ihre Carboxylgruppe an ein Polymer als
unlöslicher Träger angeknüpft. Dies geschieht entweder vorzugsweise über ein Alkali- oder Erdalkalisalz der entsprechenden
N-geschützten Aminosäure, wenn ein Chlormethyl-Harz verwendet
wird, oder durch Aktivierung der Carboxylfunktion der entsprechenden N-geschützten Aminosäure, beispielsweise mit N,N'-
Dicyclohexylcarbodiimid, wenn ein Hydroxymethyl-Harz verwendet
wird. Anschließend erfolgt der schrittweise Aufbau des Peptids
durch stufenweisen Anbau der einzelnen Aminosäuren an das N-
Ende des Peptidfragments (siehe Abb. 1) und anschließende Abspaltung des verwendeten Aminoschutzrestes. Die Anknüpfung der
nächstfolgenden N-geschützten Aminosäure an die nun freie und
reaktionsfähige endständige Aminogruppe des Peptidfragments erfolgt durch Aktivierung ihrer Carboxylfunktion oder über einen reaktiven Ester. Nach erfolgtem Aufbau der Sequenz wird das Peptid vom Harz abgespalten. Die Anwesenheit der Aminosäuren Asparaginsäure, Serin, Threonin und Histidin erfordert hierbei zusätzlichen Schutz der Seitenfunktionen, z.B. durch ihre Überführung in die entsprechenden O-Benzylthreonin-, O-Benzylserin-, N-im-2,4-Dinitrophenyl-histidin- und Asparaginsäure-ß-tert.-butylester-Derivate.

Bei der erfindungsgemäßen Festphasen-Synthese wird vorzugsweise ein quellbares Polymer, z.B. chlormethyliertes Polystyrol, das vorzugsweise mit 1 % Divinylbenzol quervernetzt ist, verwendet (PS-1 % DVB).

Besonders vorteilhaft wird erfindungsgemäß das Verfahren in der Weise durchgeführt, daß Glycin als erste Aminosäure in seiner geschützten Form, wie z.B.

Boc - Gly -OH

mit Hilfe seines Cäsium-Salzes, das man zweckmäßigerweise durch Umsetzung der oben erwähnten geschützten Aminosäure mit Cäsiumcarbonat oder Cäsiumhydroxid erhält, an chlormethyliertes Polystyrol in einem Lösungsmittel, vorzugsweise in einem aprotischen Lösungsmittel wie Dimethylformamid, verestert wird. Anschließend wird die verwendete Aminoschutzgruppe abgespalten, z.B. die N-tert.Butoxycarbonylgruppe mit einer Säure, z.B. mit 50%iger Trifluoressigsäure in Dichlormethan, vorzugsweise nach Vorwaschen mit Dichlormethan und die Fmoc-Gruppe mit Diethylamin in Dimethylformamid.

Pro anschließendem Synthese-Zyklus wird mit einem Überschuß der entsprechenden Boc- oder Fmoc-Aminosäure bzw. deren aktiviertem Ester, z.B. mit der 3- bis 6-fachen Menge, gegebenenfalls in Gegenwart eines Kupplungsreagenzes wie N,N'-Dicyclohexyl-carbodiimid gekuppelt, wobei dieser Vorgang mehrmals, z.B. 2 bis 5 mal, ohne vorherige Prüfung auf noch gegebenenfalls vorhandene freie Aminogruppen mit einem weiteren vergleichbaren Überschuß der entsprechenden Boc- oder Fmoc-Aminosäure und Kupplungsreagenz oder deren aktiviertem Ester wiederholt wird (Nachkupplung). Nach Vorwaschen wird der Boc- oder Fmoc-Schutzrest wie vorstehend beschrieben abgespalten.

Dieser Synthese-Zyklus wird gemäß Abb. 1 solange mit den entsprechenden N-geschützten Aminosäuren bzw. deren reaktiven Estern wiederholt, bis das gewünschte geschützte Dekapeptid-Harz erhalten ist.

Das Aminosäure-Derivat Boc-Gln wird hierbei vorzugsweise mit Hilfe eines seiner reaktiven Ester, z.B. über seinen p-Nitrophenylester, gekuppelt. Desweiteren kann bei den Kupplungen und den Nachkupplungen 1-Hydroxy-benzotriazol als Katalysator zugesetzt werden.

Nach jedem Synthese-Zyklus wird mit Essigsäureanhydrid/N-Methylmorpholin acetyliert, um eine gegebenenfalls bei der Kupplung nicht umgesetzte Aminogruppe des Peptidfragments für die nächste Kupplung zu blockieren. Außerdem wird nach der 9. Kupplung der Fmoc-Schutzrest mit einer Base, z.B. mit Diethylamin, abgespalten.

Das partiell geschützte Dekapeptidhydrazid erhält man nach durchgeführter Synthese durch Zugabe von Hydrazin zu dem voll geschützten Peptidharz, suspendiert in Dimethylformamid. Zur weiteren Reinigung wird dieses umgefällt, z.B. aus DMF/Ether, wobei die gelben Dnp-Produkte weitgehend entfernt werden, über eine Gelsäule, z.B. Sephadex LH2O, mit Methanol chromatographiert, einer Reversed-phase-Chromatographie an Kieselgel, z.B. Kieselgel RP-8, mit Methanol/Wasser (90/10) unterworfen und aus Methanol/Ether umgefällt.

Das schutzgruppenfreie Dekapeptid erhält man nach durchgeführter Synthese durch Zugabe von Bromwasserstoff/Eisessig in die Suspension des Peptidharzes in Trifluoressigsäure unter Zugabe von Resorcin und Thioanisol. Boc-, Bzl- und O-Bu$^t$-Reste werden gleichzeitig entfernt. Nach Behandlung des so erhaltenen N-im-Dnp-His-Dekapeptids mit Mercaptoethanol und anschließender Gelchromatographie, z.B. Sephadex LH2O, und Reversed-phase-Chromatographie, z.B. an Kieselgel RP-8, erhält man das freie Dekapeptid.

Das nachstehende Beispiel soll die Erfindung näher erläutern ohne sie zu beschränken. Die Synthese des Dekapeptids kann natürlich auch unter Verwendung anderer in der Peptidchemie üblicher Kondensationsverfahren und anderer üblicher Schutzgruppen in ähnlicher Weise durchgeführt werden:

Beispiel für die Festphasen-Synthese:

I. Herstellung des Dekapeptids am Träger:

Stufe 1

Veresterung von N-tert.Butyloxycarbonyl-glycin mit chlormethyliertem Polystyrol:

15 g trockenes PS-1% DVB und 12,3 g (40 mmol) N-tert.Butoxycarbonyl-glycin-Cäsiumsalz werden 28 Stunden bei 50°C in DMF gerührt. Anschließend wird je dreimal mit DMF, DMF/Wasser (9:1) und Ethanol gewaschen und über Phosphorpentoxid im Vakuum getrocknet. Die Beladungsbestimmung ergab 0,4 mmol Gly/g.

Stufe 2

Nach Abspaltung der Boc-Gruppe mit Dichlormethan/Trifluoressigsäure (1:1) (1 x 2 Minuten, 1 x 5 Minuten Reaktionsdauer) und Neutralisation mit Triethylamin in Chloroform

- 6 -

0051204

(1:5) (1 x 2 Minuten, 1 x 5 Minuten) erfolgt die Kupplung mit 7,5 (30 mmol) BOC-Leu-OH x $H_2O$ (vorgetrocknet) und die Nachkupplung mit 7,5 g (30 mmol) BOC-Leu-OH x $H_2O$ analog Stufe 3.

<u>Stufe 3</u>

Nach Schutzgruppenabspaltung und Neutralisation analog Stufe 2 werden 5,9 g (20 mmol) BOC-Ser(Bzl)-OH in 60 ml Dichlormethan gelöst und zum Dipeptid-Harz gegeben. Dann werden 20 ml (20 mmol) einer 1 molaren Lösung von DCC in Dichlormethan zugefügt. Nach 45 Minuten werden zur 1. Nachkupplung 5,9 g (20 mmol) Boc-Ser(Bzl)-OH in 60 ml Dichlormethan und 20 ml DCC-Lösung (1 molar in Dichlormethan) zugefügt. Nach 40 Minuten wird 2 x mit Methanol/Dichlormethan 1 : 4, 2 mal mit Dichlormethan, 1 mal mit 10 % Triethylamin in Dichlormethan und 3 mal mit Dichlormethan gewaschen. Zur 2. Nachkupplung werden 5,9 g (20 mmol) Boc-Ser(Bzl)-OH in 60 ml Dichlormethan, 20 mmol HOBt und 20 ml DCC-Lösung (1 molar in Dichlormethan) zugefügt und 60 Minuten reagieren lassen. Dann wird 3 mal mit DMF gewaschen. Anschließend wird 15 Minuten mit 6 ml Essigsäureanhydrid in 40 ml DMF und 3,3 ml N-Methylmorpholin in 40 ml DMF reagieren lassen, dann 3 mal mit DMF, 3 mal mit Methanol/Dichlormethan 1:4, 3 mal mit Ethanol und 3 mal mit Dichlormethan gewaschen.

<u>Stufe 4</u>

Kupplung mit 9,6 g (20 mmol) BOC-His(Dnp)-OH, Nachkupplung mit 9,6 g (20 mmol) BOC-His(Dnp)-OH analog Stufe 3. Boc-His-(Dnp)-OH muß vor Zusatz des Dichlormethans in etwas DMF gelöst werden.

<u>Stufe 5</u>

Kupplung mit 6,18 g (20 mmol) BOC-Thr(Bzl)-OH, Nachkupplung mit 6,18 g (20 mmol) BOC-Thr(Bzl)-OH analog Stufe 3.

## Stufe 6

Nach Abspaltung der Boc-Schutzgruppe und Neutralisation analog Stufe 2 werden 22,0 g (60 mmol) BOC-Gln-ONp in 60 ml DMF gelöst und zum Peptid-Harz gegeben. Nach 1/2 Stunde werden 8,1 g (60 mmol) HOBt zugefügt und weitere 11 Stunden reagieren lassen. Dann wird 3 mal mit DMF, 3 mal mit Methanol und 2 mal mit DMF gewaschen. Dann wird 15 Minuten mit 6 ml Essigsäureanhydrid und 3,3 ml N-Methylmorpholin in 60 ml DMF behandelt. Anschließend wird 2 mal mit DMF, 3 mal mit Methanol und 3 mal mit Dichlormethan gewaschen.

## Stufe 7

Kupplung mit 4,3 g (20 mmol) Boc-Pro-OH, 1. Nachkupplung mit 4,3 g (20 mmol) Boc-Pro-OH, 2. Nachkupplung mit 4,3 g (20 mmol) Boc-Pro-OH analog Stufe 3.

## Stufe 8

Kupplung mit 7,5 g (30 mmol) Boc-Leu-OH x $H_2O$, Nachkupplung mit 7,5 g (30 mmol) Boc-Leu-OH x $H_2O$ analog Stufe 3.

## Stufe 9

Kupplung mit 12,34 g (30 mmol) FMOC-Asp(Bu$^t$)-OH, 1. Nachkupplung mit 12,34 g (30 mmol) FMOC-Asp(Bu$^t$)-OH, 2. Nachkupplung mit 8,23 g (20 mmol) FMOC-Asp(Bu$^t$)-OH analog Stufe 3. Die Fmoc-Gruppe wird mit Diethylamin/DMF (1:9) in 30 Minuten Reaktionszeit abgespalten.

## Stufe 10

Kupplung mit 5,9 g (20 mmol) Boc-Ser(Bzl)-OH, 1. Nachkupplung mit 5,9 g (20 mmol) Boc-Ser(Bzl)-OH, 2. Nachkupplung mit 5,9 g (20 mmol) Boc-Ser(Bzl)-OH analog Stufe 3.

II. Abspaltung des Peptids vom Träger mit Hydrazin:

5 g Peptidharz werden in einer Lösung von 2,5 g Hydraziniumhydroxid in 40 ml DMF 20 Stunden gerührt. Dann wird die Lösung des partiell geschützten Dekapeptidhydrazids abgesaugt und das Harz dreimal mit insgesamt 20 ml DMF nachgewaschen. Die DMF-Lösung wird unter Rühren zu wasserfreiem Ether getropft. Dabei flockt das Dekapeptidderivat aus. Es wird zweimal unter Zentrifugieren mit Ether gewaschen, wodurch sich die gelben Nebenprodukte teilweise entfernen lassen. Dann wird noch einmal in jeweils 2 ml DMF und 2 ml Methanol gelöst und wieder mit Ether gefällt.
Ausbeute: 2,91 g.

III. Reinigung des partiell geschützten Dekapeptidhydrazids durch Gel-Chromatographie

2,9 g partiell geschütztes Dekapeptidhydrazid werden in 20 ml Methanol gelöst und an Sephadex LH 20 mit Methanol als Fließmittel chromatographiert (Säule: 5 x 86 cm). Die nach 350 ml Elutionsvolumen erscheinenden Fraktionen werden im System Chloroform/Methanol/Wasser/Eisessig (65:25:4:3) auf Kieselgel-Platten untersucht (Sprühreagens: Chlor/4,4'-Bis(dimethylamino)diphenyl-methan und Hydrazid-Reagens: $K_3[Fe(CN)_6]/FeCl_3$). Die Hauptmenge an Dekapeptidhydrazid-Derivat erscheint im Elutionsvolumen 590-723 ml (Fraktionsvolumen 19 ml). Nach Vereinigung der Fraktionen und Abdampfen des Methanols werden 2,24 g partiell geschütztes Dekapeptidhydrazid erhalten, das immer noch eine Gelbfärbung zeigt. Durch diese Sephadex-LH 20-Chromatographie werden die stark hydrophilen Verunreinigungen entfernt.

IV. Reinigung des partiell geschützten Dekapeptidhydrazids durch Reversed-Phase-Chromatographie

2 g partiell geschütztes Dekapeptidhydrazid werden an Kieselgel RP-8 (Merck-Fertigsäule Lobar B, Lichroprep RP-8, Art. Nr. 11804) mit Methanol/Wasser (90:10) chromatographiert. Die gelben Nebenprodukte werden dabei später als das Peptid eluiert.

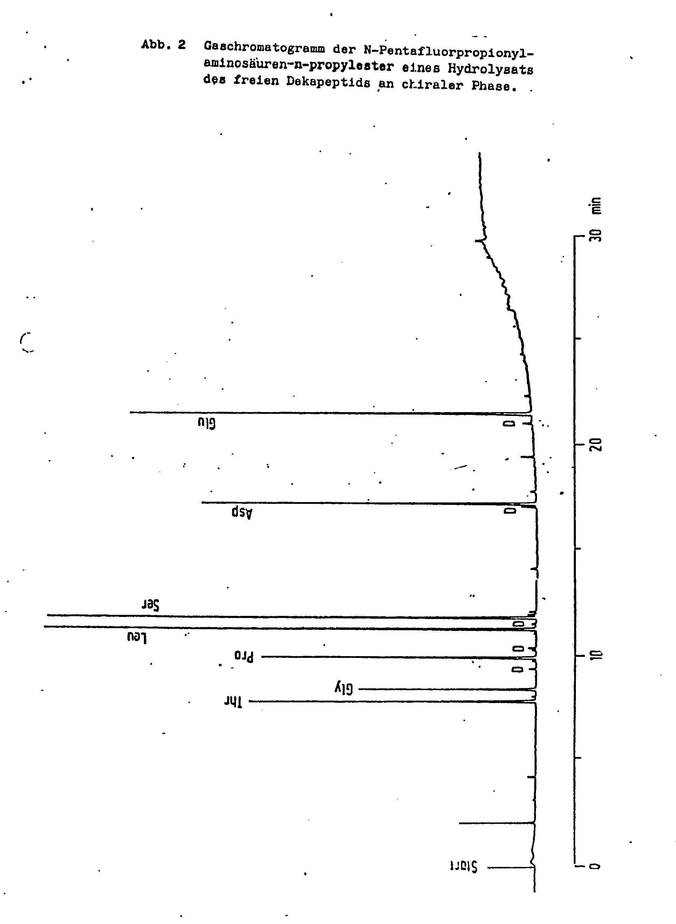

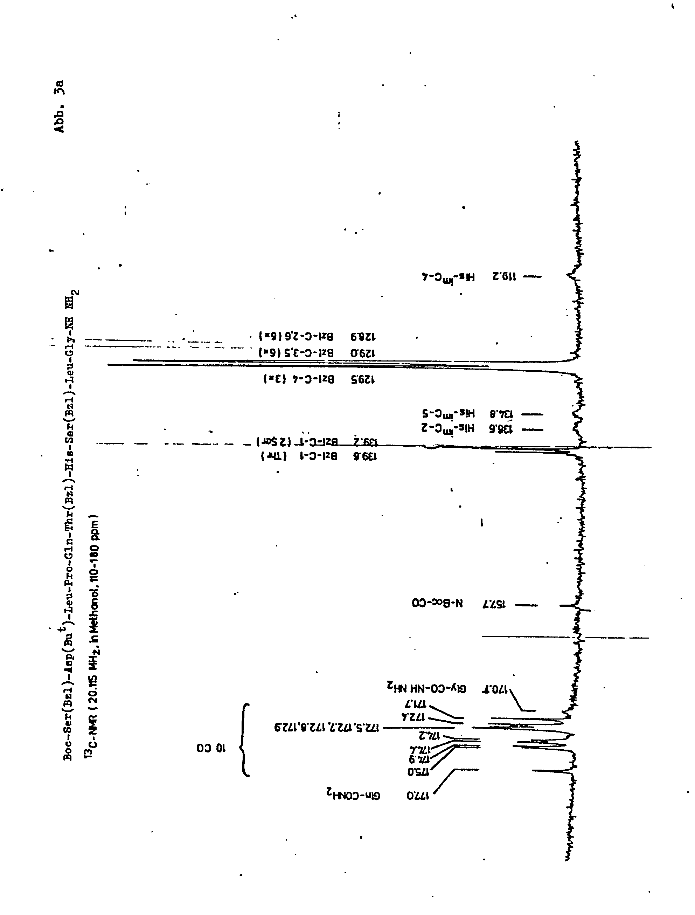

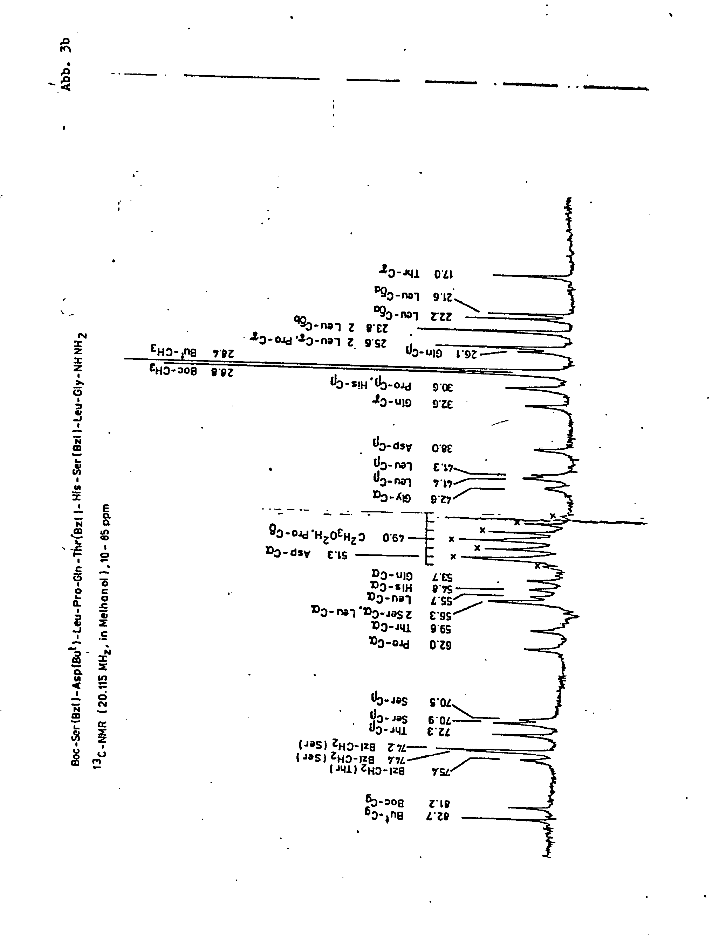

Die Identität des gemäß vorstehenden Beispiels erhaltenen partiell geschützten Dekapeptidhydrazids wird durch Aminosäurenanalyse, gaschromatographischen Racemattest (siehe Abb. 2) sowie durch $^{13}$C-NMR-spektroskopische Untersuchungen (siehe Abb. 3a und 3b) bewiesen.

Die Aminosäurenanalyse wird in einem Biotronik-Aminosäureanalysator LC 6000 E durchgeführt, die Hydrolyse erfolgt mit 6n HCL bei 110°C über 24 Stunden. Ohne Korrektur der Hydrolyseverluste wird die nachstehende Verteilung an Aminosäuren festgestellt:

Tabelle 1

| Aminosäure | Zahl der Aminosäurereste | |
|---|---|---|
| | gefunden | berechnet |
| Asp | 1,00 | 1 |
| Thr | 0,84 | 1 |
| Ser | 1,12 | 2 |
| Glu | 1,00 | 1 |
| Pro | 0,94 | 1 |
| Gly | 1,00 | 1 |
| Leu | 1,78 | 2 |
| $NH_3$ | 0,85 | 1 |
| His | 1,00 | 1 |

Die $^{13}$C-NMR-Spektren werden im NMR-Spektrometer WH-90 der Firma Bruker-Physik, Karlsruhe, vermessen, das Dekapeptidhydrazid-Derivat wurde in Methanol gelöst.

## V. Abspaltung des schutzgruppenfreien Dekapeptids vom Träger

3 g getrocknetes voll geschütztes Peptidharz werden in 10 ml Trifluoressigsäure suspendiert. Nach 10 Minuten werden 20 ml 17%ige Bromwasserstoff/Eisessig-Lösung zugegeben. Die mit etwas Thioanisol und Resorcin versetzte Reaktionsmischung wird bei Raumtemperatur im Dunkeln 2 Stunden gerührt. Nach Verdünnen mit 20 ml Eisessig wird am Rotationsverdampfer auf die Hälfte eingeengt. Nach nochmaliger Zugabe von 20 ml Eisessig wird das Harz unter Ausschluß von Feuchtigkeit abfiltriert. Das gelbe, klare Filtrat wird wieder auf etwa 10 ml eingeengt und zu 100 ml wasserfreiem Ether unter Rühren zugetropft. Die schwach gelbe Ausfällung des Peptids wird zweimal mit Ether gewaschen und dann in 10 ml Wasser gelöst. Die klare Peptid-Lösung (pH 1) wird mit Soda-Lösung auf pH 8.0 eingestellt und dann mit 2 ml Mercaptoethanol versetzt. Der danach ausgefallene Niederschlag wird mit 7 ml DMF wieder gelöst. Nach Zugabe von weiteren 2 ml Mercaptoethanol wird bei pH 8.75 eine Stunde gerührt. Dann wird mit 4 x 80 ml Ether extrahiert. Die wäßrige Phase wird mit Essigsäure auf pH 4.8 angesäuert und nochmals mit 4 x 50 ml Ether extrahiert.

## VI. Chromatographische Reinigung des freien Dekapeptids

Die oben erhaltene, braungelbe Peptidlösung wird an Sephadex G-15 mit 0,1 molare Essigsäure chromatographiert (Säule: 2.5 x 95 cm, Elutionsgeschwindigkeit 1 ml/min). Die nach 185-300 ml erscheinenden peptidhaltigen, dünnschichtchromatographisch mit dem System 1-Butanol/Eisessig/Wasser (3:1:1) detektierten Fraktionen (10 ml) werden vereinigt, lyophilisiert und ein zweites Mal chromatographiert. Die in den Fraktionen 201-275 ml enthaltene Peptid-Lösung wird eingedampft.

Ausbeute: 2,24 g

300 mg des so erhaltenen gelben Pulvers werden in 1,5 ml Methanol/Wasser (9:1) gelöst und an Kieselgel RP-8 (Lichroprep RP-8, Lobar Säule Typ C, Fa. Merck) chromatographiert (Elutionsgeschwindigkeit: 1 ml/min, Fraktionsvolumen 6 ml). Die im Elutionsvolumen von 376-424 ml enthaltene Peptidfraktion wird im Vakuum eingedampft und lyophilisiert.

Ausbeute: 174 mg; $R_f$ 0.23 (Kieselgel, System: 1-Butanol/ Eisessig/Wasser (3:1:1)).

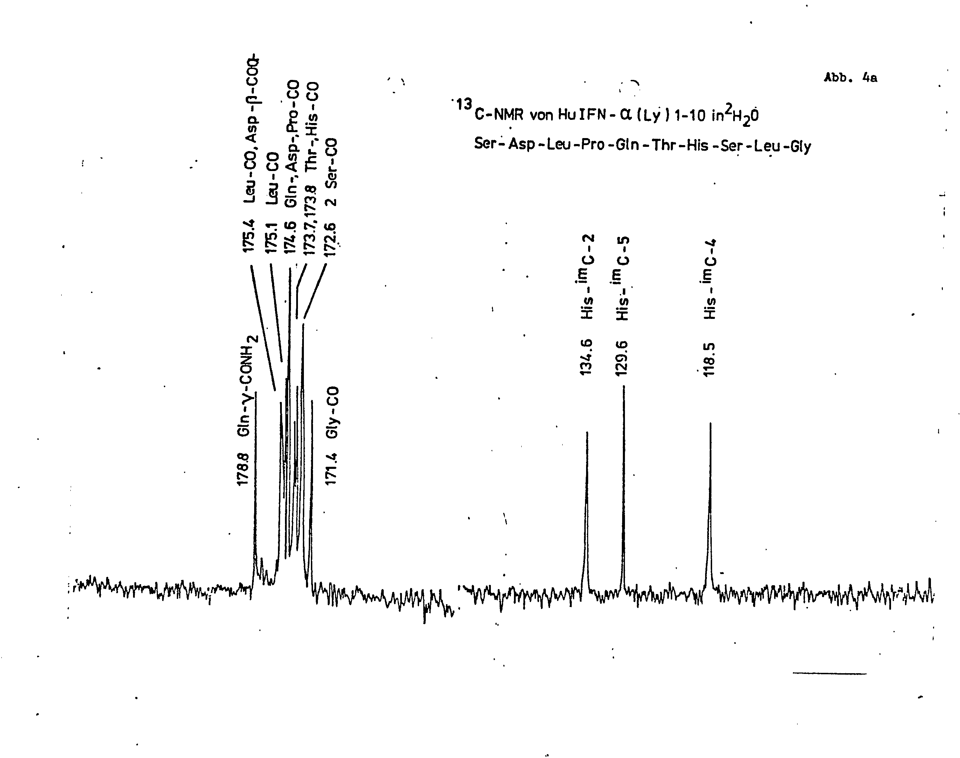

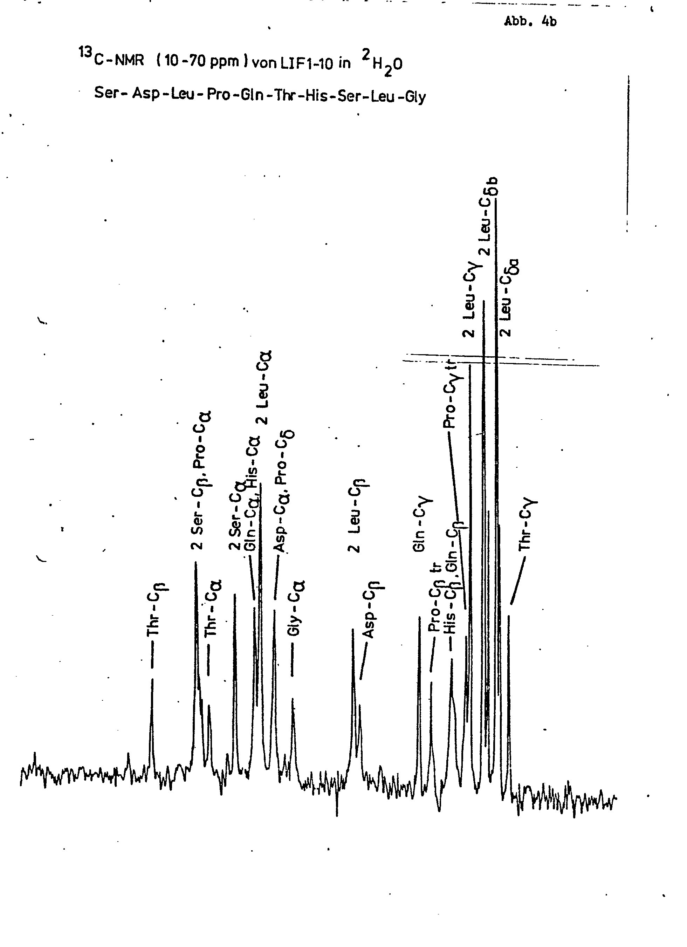

Die Identität des gemäß vorstehenden Beispiels erhaltenen Dekapeptids wurde durch $^{13}$C-NMR-spektroskopische Untersuchungen (siehe Abb. 4a und 4b) und durch Aminosäurenanalyse bewiesen.

Die Aminosäurenanalyse wird in einem Biotronik-Aminosäurenanalysator LC 600 E durchgeführt. Die Hydrolyse erfolgt mit 6 N Salzsäure bei 110°C und über 18 bis 72 Stunden. Ohne Korrektur der Hydrolyseverluste wird die nachstehende Zusammensetzung an Aminosäuren festgestellt:

Tabelle 2

| Aminosäure | Zahl der Aminosäurereste | |
|---|---|---|
| | gefunden | berechnet |
| Asp | 1,06 | 1 |
| Thr | 0,99 | 1 |
| Ser | 1,76 | 2 |
| Glu | 1,15 | 1 |
| Pro | 1,06 | 1 |
| Gly | 1,00 | 1 |
| Leu | 1,94 | 2 |
| His | 1,00 | 1 |

Die gaschromatographische Untersuchung der N-Pentafluorpropionylaminosäure-n-propylester des Totalhydrolysats des Dekapeptids an der chiralen Phase Chirasil-Val (J. Chromatogr. 146, 197 (1978)) erwies eine sehr hohe enantiomere Reinheit der Aminosäurereste des Dekapeptids (siehe Abb. 2).

Die $^{13}$C-NMR-Spektren werden im NMR-Spektrometer WH-90 der Firma Bruker-Physik, Karlsruhe, bei $30^{\circ}$C vermessen, Lösungsmittel: $^{2}$H$_2$O (siehe Abb. 4a und 4b).

Das neue Dekapeptid und sein Hydrazid können als Haptene eingesetzt werden, die mit einem natürlichen Protein, wie z.B. Humanserumalbumin, Rinderserumalbumin, Eialbumin oder mit einem synthetischen Polypeptid wie z.B. poly-L-Lysin, poly-L-Alanyl-L-lysin oder anderen Trägern wie z.B. modifizierte Dextrane nach bekannten Methoden gekuppelt werden. Das Peptid läßt sich auch über andere Polyamine, wie z.B. 1,6-Diaminohexan, verknüpfen. Ein so hergestelltes Immunogen oder das Dekapeptid selbst können mit bekannten Methoden zur Erzeugung von Antiseren bzw. Antikörpern gegen menschliches Lymphoblasteninterferon eingesetzt werden.

Das neue Dekapeptid kann an Stelle des menschlichen Lymphoblasteninterferons in geeigneter Zubereitung zur Therapie verwendet werden.

Das neue Dekapeptid ist als Tracer für die immunologische Bestimmung von menschlichem Lymphoblasteninterferon geeignet. Dazu kann es einerseits direkt nach bekannten Methoden, wie z.B. mit radioaktivem Jod oder anderen geeigneten Markern, z.B. mit Enzymen wie Peroxydasen oder mit fluoreszierenden Verbindungen, markiert werden. Andererseits läßt es sich auch in Form der oben erwähnten Kupplungsprodukte mit den gleichen oder ähnlichen Methoden markieren und als Tracer verwenden.

Das neue Dekapeptid eignet sich ferner in Form eines Kupplungsproduktes mit einem immobilen Träger, wie z.B. Dextranen, Sepharose oder Polystyrol, oder modifizierten anorganischen Trägern, wie z.B. Biogel oder CPG-10, zur Isolierung und Reinigung von Antikörpern gegen menschliche Lymphoblasteninterferone. Derart gewonnene Antikörper gestatten in bekannter Weise die Reinigung und Isolierung von menschlichem Lymphoblasteninterferon.

Das nach der schrittweisen Synthese am Träger befindliche voll geschützte Dekapeptid kann auch durch Verseifen oder Umesterung vom Träger abgespalten werden. Die abgespaltenen partiell bzw. voll geschützten Dekapeptidderivate stellen außerdem wertvolle Zwischenprodukte für die Synthese von höheren Peptiden des Lymphoblasteninterferons oder des Lymphoblasteninterferons selbst dar.

Die nachstehenden Beispiele beschreiben verschiedene Anwendungsmöglichkeiten des Dekapeptids und seines Hydrazids:

## Beispiel A

### Kupplungen mit poly-L-Lysin

a) Mit m-Xylylendiisocyanat

79 mg (62,5 nmol) partiell geschütztes Dekapeptidhydrazid werden in 3 ml Dioxan gelöst und mit 1 ml Wasser pH 7 versetzt. Dazu werden unter Rühren 30 $\mu$l (ca. 200 nmol) m-Xylylendiisocyanat gegeben. Nach 3 Minuten wird eine Lösung von 50 mg Poly-L-lysin-hydrobromid (M. G. 37 300) in 1 ml Wasser (pH 9.5, mit 1 N Natronlauge eingestellt) zugegeben. Nach 4 Stunden bei Raumtemperatur werden ca. 20 ml Wasser zugegeben und mit Eisessig ca. pH 7 eingestellt. Anschließend wird im Vakuum zur Hälfte eingeengt und nach Zugabe von 20 ml Wasser lyophilisiert.
Ausbeute: 170 mg.

100 mg des über Phosphorpentoxid getrockneten Konjugats werden mit 2 ml Trifluoressigsäure versetzt. Nach 10 Minuten werden zu dieser Suspension 8 ml 40%iger Bromwasserstoff in Eisessig (versetzt mit je ca. 30 mg Resorcin und Thioanisol) zugegeben und 30 Minuten im Dunkeln bei Raumtemperatur gerührt. Nach dem Einengen im Vakuum wird das Konjugat durch Einrühren der Eisessig-Lösung in absoluten Ether (ca. 100 ml) ausgefällt. Nach 2-maligem Waschen mit je 100 ml Ether im Zentrifugenglas wird der Niederschlag (180 mg) in Wasser gelöst und gegen 1 N Essigsäure 24 Stunden und anschließend gegen dest. Wasser 24 Stunden dialysiert (Ausschlußgrenze 8 000). Nach Zentrifugation des Dialysats erhält man 31 mg wasserunlösliches Konjugat der Beladung 6.4 Mol Dekapeptidhydrazid pro Mol poly-L-Lysin. Aus der wäßrigen Lösung lassen sich nach deren Lyophilisation 1,7 mg lösliches Konjugat der Beladung 42:1 erhalten.

b) Durch Azidkupplung

70 mg (50 μmol) partiell geschütztes Dekapeptidhydrazid werden in 1,5 ml DMF gelöst und bei -20°C mit 70 μl (126 μmol = 2.5 Äquivalente) 1.8 N HCl/Essigsäureethylester versetzt. Dann werden bei -20°C zuerst 8 μl (60 μmol = 1.2 Äquivalente) Isoamylnitrit und nach 5 Minuten bei -10°C weitere 2 μl Isoamylnitrit zugefügt bis zur Blaufärbung mit Kaliumjodid/Stärke-Papier). Nach 10 Minuten bei -10°C wird eine eiskalte Lösung von 70 mg poly-L-Lysin-hydrobromid in 0,5 ml Wasser (mit 4 μl Triethylamin auf pH 9 eingestellt) zugegeben. Dabei tritt eine Trübung der Lösung ein. Nach 20 Minuten wird mit weiteren 6 μl Triethylamin auf pH 8,5 eingestellt und die stark trübe, viskose Lösung bei -10°C 30 Minuten gerührt und dann bei +4°C 18 Stunden aufbewahrt. Es wird noch bei Raumtemperatur 8 Stunden gerührt, dann mit 10%iger Essigsäure auf pH 6 gestellt und nach Zugabe von viel tert.Butanol und etwas Wasser lyophilisiert. Zur Abspaltung der Schutzgruppen wird wie unter a) verfahren. Man erhält 36,5 mg gut wasserlösliches, farbloses Konjugat mit einer Beladung von 14,9 Mol Dekapeptid pro Mol poly-L-Lysin.


Beispiel B


Jodmarkierung des Kupplungsprodukts des Dekapeptids bzw. seines Hydrazids mit poly-L-Lysin


Von dem getrockneten Kupplungsprodukt werden 2 mg eingewogen und in 25 µl Wasser gelöst. Diese Lösung wird mit 0,1 mM Natrium-Borat-Puffer pH 9 verdünnt bis die Konzentration des Kupplungsproduktes 10 µg/µl beträgt. 12 µl davon mit einer Konzentration von Borat von 0,1 M pH 9,0 und vom Kupplungsprodukt mit 10 µg/µl werden bei einer Temperatur von $0^{o}$C (auf Eis) in ein Reaktionsgefäß gefüllt, in welchem das BOLTON-HUNTER-Reagenz (N-Hydroxy-succinimidester der mit $^{125}$J-jodierten p-Hydroxyphenylpropionsäure) aus Benzol getrocknet war.


Die Reaktion wird nach einer Stunde gestoppt durch Zugabe von 90 µl 0,3 M Glycin in 0,1 M Natrium-Borat, pH 9,0. Nach weiteren 10 Minuten wird das gesamte Volumen nun auf eine Chromatographiesäule gegeben (7 ml Bettvolumen, Sephadex G 25 Medium (Pharmacia, Uppsala), welche mit 50 mM Natriumphosphat pH 7,5 und 0,25 % Gelatine äquilibriert worden war und in diesem Puffer auch entwickelt wird. Die hochmolekulare markierte Substanz wird hierbei von niedrigmolekularen radioaktiven Reaktionsprodukten getrennt und erscheint in den ersten Fraktionen des Voidvolumens.


Eine bessere Ausbeute - verglichen mit diesen ursprünglichen Vorschriften von BOLTON und HUNTER - wird erreicht, wenn nach dem Ende der Reaktion die Lösung auf einen sauren pH gebracht wird. Nach der Zugabe von Glycin wird der pH von 9 auf 4 eingestellt, durch eine weitere Zugabe von 100 µl 20 mM Natrium-Acetat pH 4,0. Die Chromatographiesäule ist entsprechend mit 20 mM Natrium-Acetat pH 4,0 und 0,25 % Gelatine vorbehandelt und wird in diesem Puffer entwickelt.

Ausgehend von einer Radioaktivität von 0,2 mCi eines BOLTON-HUNTER-Reagenzes mit der spezifischen Radioaktivität von 2000 Ci/mmol wird eine Markierung von mindestens $3 \times 10^5$ cpm (counts per minutes)/µg Kupplungsprodukt erreicht.

Beispiel C

Kupplung an AH-Sepharose 4B zur Herstellung von Materialien zur Affinitätschromatographie

35 mg (25 µmol) partiell geschütztes Dekapeptidhydrazid werden in 2 ml Trifluoressigsäure gelöst und mit 4 ml 33%ige Bromwasserstoffsäure/Eisessig versetzt. Nach einer Stunde wird am Rotationsverdampfer eingeengt, der Rückstand in 2 ml Eisessig aufgenommen und die Lösung unter Rühren zu 50 ml wasserfreiem Ether getropft. Der Niederschlag wird abzentrifugiert und über festem Kaliumhydroxid im Vakuum getrocknet. Das erhaltene ungeschützte Dekapeptidhydrazid-dihydrobromid wird analog Beispiel A(b) in das Azid überführt und in DMF/Wasser an die Aminogruppen von 300 mg AH-Sepharose 4B gekuppelt. Nach 24 Stunden Reaktionszeit wird das AH-Sepharose 4B-Dekapeptid solange mit DMF, Dioxan und Wasser gewaschen, bis keine niedermolekularen Bestandteile im Dünnschichtchromatogramm nachweisbar sind.

Beispiel D

Kupplung des Dekapeptids an Lysin-Sepharose 4B zur Affinitätschromatographie

35 mg partiell geschütztes Dekapeptidhydrazid werden, wie unter Beispiel C beschrieben, mit Bromwasserstoff/Trifluoressigsäure behandelt und in das Azid überführt, das an 300 mg Lysin-Sepharose 4B (Pharmacia) gekuppelt wird.

## Beispiel E

Herstellung von Kupplungsprodukten des Dekapeptids an Aminopropyl-CPG-10 zur Affinitätschromatogrphie

500 mg Controlled-Pore Glass CPG 10 (Porengröße 75 und 120 Å, belegt mit Aminopropylgruppen) werden mit dem aus 70 mg partiell geschütztem Dekapeptidhydrazid erhaltenen Azid analog Beispiel C gekuppelt.

## Beispiel F

Verknüpfung von zwei Molekülen Dekapeptidhydrazid über 1,6-Diaminohexan

70 mg (50 $\mu$mol) partiell geschütztes Dekapeptidhydrazid werden wie unter Beispiel A(b) beschrieben in das Azid überführt. Zu dem Azid werden bei -10$^{\circ}$C 2,9 mg (25 umol) 1,6-Diaminohexan in 200 $\mu$l DMF gegeben. Nach Einstellen auf ca. pH 8.5 mit Triethylamin wird 2 Stunden bei -10$^{\circ}$C gerührt, 24 Stunden bei 4$^{\circ}$C stehengelassen und nochmals 8 Stunden bei Raumtemperatur gerührt. Anschließend wird die Reaktionslösung unter Rühren in wasserfreien Ether getropft. Der ausgefallene Niederschlag wird 2 x mit Ether gewaschen und über Diphosphorpentoxid/Kaliumhydroxid getrocknet. Dann wird in Trifluoressigsäure gelöst und mit 4 ml 33%igem Bromwasserstoff/Eisessig versetzt. Das schutzgruppenfreie Produkt wurde nach 2 Stunden mit Ether ausgefällt, über Kaliumhydroxid getrocknet und anschließend in DMF über Sephadex LH 20 chromatographiert (Säule 1 x 90 cm). Die das Dimere enthaltenden, zuerst eluierten Fraktionen (dünnschichtchromatographische Detektion) werden im Vakuum zur Trockne eingeengt.

Patentansprüche
=================================

1. Neues Dekapeptid der Formel H-Ser-Asp-Leu-Pro-Gln-Thr-His-Ser-Leu-Gly-OH

2. Neues Dekapeptidhydrazid der Formel

   $Boc-Ser(Bzl)-Asp(Bu^t)-Leu-Pro-Gln-Thr(Bzl)-His-Ser(Bzl)-$
   $Leu-Gly-NHNH_2$

3. Verwendung des Dekapeptids gemäß Anspruch 1 als Hapten,
   Tracer oder Antikörper.

4. Neues Dekapeptidhydrazid der Formel

   $H-Ser-Asp-Leu-Pro-Gln-Thr-His-Ser-Leu-Gly-NH-NH_2$

5. Verwendung des Dekapeptids gemäß Anspruch 4 als Hapten,
   Tracer oder Antikörper.

6. Verwendung des Dekapeptidhydrazids gemäß Anspruch 2 zur
   Herstellung von höheren Peptiden des Lymphoblasteninterferons
   oder des Lymphoblasteninterferons.

7. Verfahren zur Herstellung des Dekapeptids der Formel H-Ser-Asp-Leu-Pro-Gln-Thr-His-Ser-Leu-Gly-OH oder des Dekapeptidhydrazids der Formel

   $Boc-Ser(Bzl)-Asp(Bu^t)-Leu-Pro-Gln-Thr(Bzl)-His-Ser(Bzl)-$
   $Leu-Gly-NH-NH_2$

dadurch gekennzeichnet, daß man in einem Lösungsmittel die C-terminale geschützte Aminosäure an ein Polymer als unlöslichen Träger anknüpft,

nach jeweiliger Abspaltung des Aminoschutzrestes das Peptid durch stufenweisen Anbau der einzelnen gegebenenfalls in der Seitenfunktion geschützten N-geschützten Aminosäuren an das jeweilige Aminoende des Peptidfragments am polymeren Träger gemäß obiger Formel aufbaut, wobei man vor jedem neuen Synthese-Zyklus gegebenenfalls noch vorhandene freie Aminogruppen durch Acetylierung blockiert und anschließend die verwendete Aminoschutzgruppe abspaltet,

und anschließend von dem so erhaltenen voll geschützten Peptid-Harz mit Hydrazin das partielle geschützte Dekapeptidhydrazid oder mit Bromwasserstoff/Eisessig das Dekapeptid abspaltet und das so erhaltene Dekapeptidhydrazid oder das Dekapeptid nach an sich bekannten Methoden reinigt.

8. Verfahren gemäß Anspruch 7, dadurch gekennzeichnet, daß man als Aminoschutzgruppe die tert.Butyloxycarbonyl- oder 9-Fluorenylmethyloxycarbonylgruppe verwendet.

9. Verfahren gemäß Anspruch 7, dadurch gekennzeichnet, daß man die Seitenfunktionen der Aminosäuren Asparaginsäure, Serin, Threonin und Histidin durch Überführung in entsprechende O-Benzylserin, O-Benzylthreonin-, N-im-2,4-Dinitrophenyl-histidin- und Asparaginsäure-ß-tert.butylester-Derivate schützt.

10. Verfahren gemäß Anspruch 7, dadurch gekennzeichnet, daß man Glycin als C-terminale Aminosäure in seiner geschützten Form über das Cäsium-Salz an chlormethyliertes Polystyrol anknüpft.

11. Verfahren gemäß Anspruch 7, dadurch gekennzeichnet, daß man die 2. bis 5. und 7. bis 10. N-geschützte Aminosäure durch Aktivierung der Carboxylfunktion mittels eines Kupplungsreagenzes wie N,N'-Dicyclohexylcarbodiimid anknüpft.

12. Verfahren gemäß Anspruch 7, dadurch gekennzeichnet, daß man die 6. N-geschützte Aminosäure über einen aktivierten Ester, z.B. über ihren p-Nitrophenylester, anknüpft.

13. Verfahren gemäß den Ansprüchen 7 bis 12, dadurch gekennzeichnet, daß man jeden Synthese-Zyklus mit einem Überschuß, z.B. mit der 3- bis 6-fachen Menge, der jeweiligen N-geschützten Aminosäure durchführt und dies 2 bis 5 mal wiederholt.

14. Verfahren gemäß den Ansprüchen 7 bis 12, dadurch gekennzeichnet, daß man den Boc-Schutzrest durch Behandeln mit 50%iger Trifluoressigsäure in Dichlormethan abspaltet.

15. Verfahren gemäß den Ansprüchen 7 bis 12, dadurch gekennzeichnet, daß man den Fmoc-Schutzrest durch Behandeln mit Diethylamin in Dimethylformamid abspaltet.

16. Verfahren gemäß den Ansprüchen 7 bis 15, dadurch gekennzeichnet, daß die Anknüpfung der geschützten Aminosäuren in Gegenwart eines Katalysators, z.B. in Gegenwart von 1-Hydroxy-benzotriazol, durchgeführt wird.

Boc-Gly-O$^\ominus$ Ca$^\oplus$ + CL-CH$_2$-(PS)

↓ DMF

Boc-Gly - O -CH$_2$-(PS)

↓↓↓↓ a) CF$_3$COOH/CH$_2$Cl$_2$
        b) Boc-AS-OH DCC/HOBt

Boc-Thr(Bzl)-His(Dnp)-Ser(Bzl)-Leu-Gly - O -CH$_2$-(PS)

↓ a) CF$_3$COOH/CH$_2$Cl$_2$
  b) Boc-Gln-ON$_B$,HOBt

Boc-Gln-Thr(Bzl)-His(Dnp)-Ser(Bzl)-Leu-Gly - O -CH$_2$-(PS)

↓↓↓ a) CF$_3$COOH/CH$_2$Cl$_2$
   b) Boc-AS-OH bzw. Fmoc-AS-OH DCC,HOBt

Fmoc-Asp(Bu$^t$)-Leu-Pro-Gln-Thr(Bzl)-His(Dnp)-Ser(Bzl)-Leu-Gly - O -CH$_2$-(PS)

↓

Boc-Ser(Bzl)-Asp(Bu$^t$)-Leu-Pro-Gln-Thr(Bzl)-His(Dnp)-Ser(Bzl)-Leu-Gly - O -CH$_2$-(PS)

↓ N$_2$H$_4$, DMF

Boc-Ser(Bzl)-Asp(Bu$^t$)-Leu-Pro-Gln-Thr(Bzl) - His - Ser(Bzl)-Leu-Gly -NH NH$_2$

1. HBr/CF$_3$COOH

2. HS-CH$_2$CH$_2$-OH

H - Ser - Asp- Leu-Pro-Gln - Thr - His - Ser - Leu-Gly -OH ◄

Abb. 1   Schema der Festphasensynthese zur Herstellung des partiell geschützten Dekapeptidhydrazids und des schutzgruppenfreien Dekapeptids.

1/6

0051204

Abb. 2    Gaschromatogramm der N-Pentafluorpropionyl-
aminosäuren-n-propylester eines Hydrolysats
des freien Dekapeptids an chiraler Phase.

Boc-Ser(Bzl)-Asp(Bu$^t$)-Leu-Pro-Gln-Thr(Bzl)-His-Ser(Bzl)-Leu-Gly-NH NH$_2$

$^{13}$C-NMR ( 20.115 MH$_z$, in Methanol, 110-180 ppm)

Abb. 3a

10 CO

Gln-CONH$_2$

177.0
175.0
172.9
172.4
172.2
172.5, 172.7, 172.8, 172.9
172.4
171.7
170.7  Gly-CO-NH NH$_2$

157.7  N-Boc-CO

139.6  Bzl-C-1  (Thr)
139.2  Bzl-C-1 (2 Ser)
138.6  His-$^{im}$C-2
134.8  His-$^{im}$C-5

129.5  Bzl-C-4 (3×)
129.0  Bzl-C-3,5 (6×)
128.9  Bzl-C-2,6 (6×)

119.2  His-$^{im}$C-4

Boc-Ser(Bzl)-Asp(Bu$^t$)-Leu-Pro-Gln-Thr(Bzl)-His-Ser(Bzl)-Leu-Gly-NHNH$_2$

$^{13}$C-NMR (20.115 MHz, in Methanol), 10-85 ppm

Abb. 3b

Abb. 4a

$^{13}$C-NMR von HuIFN-α(Ly)1-10 in $^2$H$_2$O

Ser-Asp-Leu-Pro-Gln-Thr-His-Ser-Leu-Gly

118.5  His-$^{im}$C-4

129.6  His-$^{im}$C-5

134.6  His-$^{im}$C-2

171.4 Gly-CO

172.6  2 Ser-CO

173.7,173.8  Thr-,His-CO

174.6  Gln-,Asp-,Pro-CO

175.1  Leu-CO

175.4  Leu-CO,Asp-β-COO-

178.8  Gln-γ-CONH$_2$

Abb. 4b

$^{13}$C-NMR (10-70 ppm) von LIF1-10 in $^2$H$_2$O

Ser-Asp-Leu-Pro-Gln-Thr-His-Ser-Leu-Gly

Europäisches
Patentamt

**EUROPÄISCHER RECHERCHENBERICHT**

Nummer der Anmeldung

EP 81 10 8535.6

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| P,X | Chemical Abstracts Band 94, Nr. 21 25. Mai 1981 Columbus, Ohio, USA JUNG et al. "Synthesis and carbon-13 NMR spectra of the N-terminal decapeptide of the sequence of human lymphoblast interferon" Seite 531, Spalte 2, Abstract Nr. 172633r | 1-16 |
| & | Hoppe-Seyler's Z. Physiol. Chem. Band 362, Nr. 3, 1981, Seiten 291 bis 304 | |
| | -- | |
| A | Chemical Abstracts Band 92, Nr. 11 17. März 1980 Columbus, Ohio, USA ZOON et al. "Amino terminal sequence of the major component of human lymphoblastoid interferon" Seite 443, Spalte 2, Abstract Nr. 92554j | |
| D,A & | Science (Washington, D.C.), Band 207 Nr. 4430, 1980, Seiten 527 bis 528 | |
| | ---- | |

**KLASSIFIKATION DER ANMELDUNG (Int. Cl.³)**

C 07 C 103/52
A 61 K 37/02
A 61 K 49/02
G 01 N 33/54

**RECHERCHIERTE SACHGEBIETE (Int. Cl.³)**

A 61 K 37/02
C 07 C 103/52

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 20-01-1982 | BREW |

EPA form 1503.1   06.78